# EUROPEAN PATENT APPLICATION

(11) **EP 4 230 229 A1**
(43) Date of publication of application: **23.08.2023**
(21) Application number: 23155724.0
(22) Date of filing: 09.02.2023
(51) Int. Cl.: A61L 2/10, A61L 9/20, A61L 2/232

(54) **INACTIVATION METHOD AND INACTIVATION SYSTEM**

(30) Priority: 17.02.2022 JP 2022022561
(71) Applicant: Ushio Denki Kabushiki Kaisha, Tokyo 100-8150 (JP)
(72) Inventor: NAITO, Keisuke, Tokyo, 100-8150 (JP)
(74) Representative: Tomerius, Isabel

(57) **Abstract**

Provided are an inactivation method and an inactivation system designed to produce a desired inactivating effect with increased reliability using ultraviolet light that has an extremely small influence on the human body. A method for inactivating bacteria or viruses in a space defined includes a step of irradiating of an inner wall surface, a floor surface in the space or a surface of an object disposed in the space where a specified substance displaying at least one of antibacterial and antiviral characteristics is present with ultraviolet light having a wavelength within a range of 190 nm or more and less than 240 nm.

## Description

### BACKGROUND OF THE INVENTION

### Field of the Invention

The present invention relates to an inactivation method and an inactivation system, and more particularly to a method and a system for inactivation in a defined space.

### Description of the Related Art

Bacteria (including fungi) or viruses present in spaces or on object surfaces may cause infectious diseases in humans or animals other than humans, and there is a fear that spread of such infectious diseases threatens our life. In particular, infectious diseases are likely to spread in facilities where people frequently gather, such as medical facilities, schools, and government offices and vehicles such as cars, trains, buses, airplanes, and ships, and therefore there is a need for effective means for inactivating bacteria or viruses (hereinafter may be collectively referred to as "pathogens").

A conventionally known method for inactivating pathogens is a method for irradiating pathogens with ultraviolet light. Deoxyribonucleic acid (DNA) exhibits the highest absorption characteristics around a wavelength of 260 nm. An emission spectrum of low-pressure mercury lamps shows high intensity around a wavelength of 254 nm. Therefore, a technique for killing pathogens using a low-pressure mercury lamp is widely used.

However, it is known that irradiation of a human with ultraviolet light in such a wavelength band has a risk of affecting the human body. The skin is divided into three parts from superficial to deep: the epidermis, the dermis, and the hypodermis deeper than the dermis, and the epidermis is further divided into four layers from superficial to deep: the stratum corneum, the stratum granulosum, the stratum spinosum, and the basal layer. When a human body is irradiated with ultraviolet light with a wavelength of 254 nm, the ultraviolet light passes through the stratum corneum, reaches the stratum granulosum or the stratum spinosum or, in some cases, reaches the basal layer, and is absorbed by DNA of cells present in these layers. This, as a result, causes a risk of skin cancer. Therefore, it is difficult to actively use ultraviolet light in such a wavelength band in places where a human may be present.

Patent Document 1 described below states that ultraviolet light with a wavelength of 240 nm or more is hazardous to a human body and that the degree of impact of ultraviolet light with a wavelength of less than 240 nm on a human body is lower than that of ultraviolet light with a wavelength of 240 nm or more. Further, Patent Document 1 specifically describes the results of an experiment of irradiation at wavelengths of 207 nm and 222 nm.

### Prior Art Document

### Patent Document

Patent Document 1: JP-A-2016-220684

### SUMMARY OF THE INVENTION

The inventor of the present invention has conducted an intensive study on inactivation of bacteria or viruses in a space, such as a room inside a building, a vehicle, or an aisle, where a human may be present, using ultraviolet light with a wavelength of less than 240 nm.

The present inventor has examined, for the study, an inactivating effect shown by ultraviolet light with a wavelength of less than 240 nm and has noticed that while an expected inactivating effect is produced by ultraviolet light with a wavelength of around 254 nm, an inactivating effect shown by ultraviolet light with a wavelength of 222 nm is substantially diminished in some cases.

In view of the above problem, it is an object of the present invention to provide an inactivation method and an inactivation system that is designed to produce a desired inactivating effect with increased reliability using ultraviolet light that has an extremely small influence on the human body.

An inactivation method according to the present invention is a method for inactivating bacteria or viruses in a space that is defined, and the method includes a step (A) of irradiating of an inner wall surface, a floor surface in the space or a surface of an object disposed in the space where a specified substance displaying at least one of antibacterial and antiviral characteristics is present with ultraviolet light having a wavelength within a range of 190 nm or more and less than 240 nm.

In the present specification, "antibacterial" is a characteristic of a substance that is displayed when the substance comes into contact with and is incorporated in the bacteria to inhibit a proliferation function of the bacteria, and "antiviral" is a characteristic of a substance that is displayed when the substance comes into contact with a virus to inactivate the virus.

Ultraviolet light having a wavelength within a range of 100 nm to 280 nm (also referred to as "deep ultraviolet light" or "UV-C") is readily absorbed by a stain adhering to an object surface, oxygen present in the air, and the like. The distinctiveness of this characteristic increases with a decrease in the wavelength that ultraviolet light has within the wavelength range.

If a stain adheres to a surface of an object to be treated, ultraviolet light with a wavelength of less than 240 nm, which has an extremely small influence on the human body, is absorbed by the stain adhering to the object and is less likely to reach pathogens present on the surface of the object to be irradiated. Hence, as for inactivation of pathogens using ultraviolet light with a wavelength of less than 240 nm, even when an object to be treated is irradiated with the ultraviolet light that has substantially high irradiance, some of the pathogens on the surface of the object are not irradiated with the ultraviolet light at a level of irradiance required for inactivation in some cases.

As compared to ultraviolet light with a wavelength of less than 240 nm, ultraviolet light with a wavelength longer than 240 nm is less likely to be absorbed by protein or sebum contained in the stain and thus is relatively less susceptible to effect of the stain.

It is conceivable that an object to be treated is irradiated with ultraviolet light at increased irradiance to produce an expected inactivating effect by ultraviolet light with a wavelength of less than 240 nm. However, even with ultraviolet lightthat has an extremely small influence on the human body, irradiating the human body with such ultraviolet light at high irradiance over a longtime is not desirable from the viewpoint of safety.

Actually, at the filing date of this application, ACGIH (American Conference of Governmental Industrial Hygienists) or JIS Z 8812 (Measuring methods of eye-hazardous ultraviolet radiation) specifies a threshold limit value (TLV) dependent on wavelength concerning the dose of ultraviolet irradiation to a human body per day (8 hours). In other words, in a case where ultraviolet light is used in an environment where a human is present, it is recommended to determine the radiation intensity of a light source unit and time of ultraviolet irradiation such that the integrated irradiation dose of the ultraviolet light within a predetermined time is equal to or less than the TLV. Thus, when an object to be treated is disposed particularly in a space where a human may be present and is irradiated with ultraviolet light, it is difficult to simply increase irradiance of the ultraviolet light.

It is conceivable that a stain adhering to the surface of an object to be treated is wiped in advance and then is irradiated with ultraviolet light. Unfortunately, such a method always requires an operator to perform cleaning and thus is inefficient. It is possible that a partial area where inactivation is not allowed is created due to an area left unwiped or uneven cleaning.

Ultraviolet light with a wavelength of less than 190 nm causes an oxygen molecule present in the atmosphere to be photolyzed, generating an oxygen atom (also referred to as "atomic oxygen") that displays high reactivity. The oxygen molecule and the oxygen atom react together by bonding to form ozone. It is for this reason that exposing the atmosphere to ultraviolet light with a wavelength of less than 190 nm is undesirable.

Thus, the present inventor has studied an inactivation method that combines ultraviolet light having a wavelength within a range of 190 nm or more and less than 240 nm with a specified substance that displays at least one of antibacterial and antiviral characteristics to find a method for satisfactorily inactivating pathogens on the surface of an object to be treated and simultaneously ensuring safety.

The present inventor has conducted, for the study, a verification experiment to examine an inactivating effect on the surface of an object to which a stain is adhering and has observed an effect higher than a total of an inactivating effect by the specified substance and an inactivating effect by the ultraviolet light. Details of the verification will be described later in the "DETAILED DESCRIPTION OF THE PREFERRED EMBODIMENT" section. As for a factor causing the above result, the present inventor has surmised that the specified substance is activated by the ultraviolet light and thus the specified substance exhibits an enhanced inactivating effect compared to a condition in which the specified substance is not irradiated with the ultraviolet light.

The inactivation method described above enables inactivation of bacteria or viruses on the object the surface of which a stain is adhering to by using the ultraviolet light that has an extremely small influence on the human body without unnecessarily increasing irradiance of the light on the surface of the object. Similarly to the surface of the object, the method enables inactivation of bacteria or viruses on the inner wall surface and the floor surface that a human may touch and that a stain is adhering to in the space.

In the inactivation method,
the step (A) may be a step that is performed by anchoring the specified substance to any of the inner wall surface, the floor surface, and the surface of the object to be treated in a state of being irradiated with the ultraviolet light in the space.

In the inactivation method,
the step (A) may be a step that is performed after the specified substance is anchored to any of the inner wall surface, the floor surface, and the surface of the object to be treated in the space by irradiating an area to which the specified substance is anchored with the ultraviolet light.

Conceivable methods for anchoring the specified substance in place include a method for anchoring a liquid material to the surface of the object by spraying the liquid material over, by applying the liquid material with a brush and by anchoring a film material to the surface of the object by sticking the film material on.

The inactivation method may be a method whereby
an inorganic antibacterial agent containing the specified substance supported by an inorganic compound is stuck on any of the inner wall surface, the floor surface, and the surface of the object to be treated in the space to anchor the specified substance to any of the inner wall surface, the floor surface, and the surface of the object.

In the inactivation method,
the specified substance contained in the inorganic antibacterial agent may be made of a metal ion or a metal compound that contains at least one metal component selected from the group consisting of silver, copper, zinc, platinum, nickel, cobalt, lead, iron, calcium, sodium, aluminum, and manganese.

An antibacterial agent is a member used to introduce the specified substance to the surface of the object, and antibacterial agents are generally classified into organic antibacterial agents and inorganic antibacterial agents. Further, organic antibacterial agents are classified broadly into synthetic antibacterial agents such as alcoholic or phenolic agents and natural antibacterial agents made of natural materials such as wasabi and tea. Organic antibacterial agents are characterized by high immediate effectiveness on pathogens despite a short validity period compared to inorganic antibacterial agents.

Inorganic antibacterial agents are antibacterial agents that use the antibacterial characteristic of metal ions derived from metallic materials that are primarily supported by inorganic compounds. Inorganic antibacterial agents are characterized by semipermanent continuance of the antibacterial effect despite slow manifestation of the effect compared to organic antibacterial agents.

Inactivation by radiation of ultraviolet light is often performed over a span of several minutes to several hours after a person leaves a room, after an object to be treated is touched, or in a predetermined time period, for example. Hence, when an antibacterial agent is used in the inactivation method of the present invention, an inorganic antibacterial agent is preferable to an organic antibacterial agent because of the semipermanent continuance of the effect.

It is known that metal ions such as silver ions, copper ions, and zinc ions display high antibacterial characteristics. When safety of humans and antibacterial characteristics are taken into consideration, silver ions are particularly preferable among these metal ions. Thus, the specified substance is preferably made of a metal ion or a metal compound that contains at least one metal component selected from the group consisting of silver, copper, zinc, platinum, nickel, cobalt, lead, iron, calcium, aluminum, and manganese. The specific substance is more preferably made of a metal ion or a metal compound that contains any metal component selected from silver, copper, and zinc among the metals above. The specific substance is particularly preferably made of a metal ion or a metal compound that contains a silver component.

An inactivation system according to the present invention is a system for inactivating bacteria or viruses on an object to be treated in a space that is defined, and the system includes:
an antibacterial layer formed on a surface of the object, the antibacterial layer containing a specified substance that displays at least one of antibacterial and antiviral characteristics; and
a light source device mounted on a ceiling or a wall surface in the space to emit ultraviolet light with a wavelength within a range of 190 nm or more and less than 240 nm toward the antibacterial layer on the object.

The inactivation system may include:
a human detector to detect whether or not a human or a part of a human is present between the light source device and the antibacterial layer;
a drive unit to change a light emission direction of the light source device; and
a controller to control the drive unit in response to a human detection signal output from the human detector,
wherein when the human detector detects presence of a human, the controller controls the drive unit to cause the light source device to emit the ultraviolet light in a direction that is not aimed at the object.

The inactivation system may include:
a human detector to detect whether or not a human is present between the light source device and the antibacterial layer; and
a controller to control intensity of the ultraviolet light emitted from the light source device in response to a human detection signal output from the human detector,
wherein when the human detector detects presence of a human, the controller controls the light source device to decrease the intensity of the emitted ultraviolet light or stop emission of the ultraviolet light.

As described above, concerning ultraviolet irradiation to the human body, the TLV is specified for ultraviolet light having a wavelength within a range of 190 nm or more and less than 240 nm despite an extremely small influence of such ultraviolet light on the human body. Thus, it is preferable to emit the ultraviolet light in a direction that is not aimed at the antibacterial layer if a human is present between the light source device and the antibacterial layer. It is preferable to decrease the intensity of the emitted ultraviolet light or stop emission of the light if a human is present in a range of the irradiation. The expression "detect whether or not a human is present between the light source device and the antibacterial layer" used herein is intended to also include a case in which only the head, hand or another part of the human is present between the light source device and the antibacterial layer.

Accordingly, the configuration described above enables the inactivation system to suppress irradiation of the human with the ultraviolet light as much as possible to comply with the specified TLV and thus provide improved safety.

A product covered by the present invention can provide sterilization and virus inactivation performance intrinsic to ultraviolet light without causing erythema or keratitis on the skin and eyes of a human and an animal. In particular, unlike conventional ultraviolet light sources, the product can be installed in an environment where people are present indoors and outdoors by taking advantage of the characteristic of the inactivation system of being able to be used in such an environment to irradiate the entire environment and provide virus inhibition and bacteria elimination in the air and on a surface of parts installed in the environment.

This accords with Goal 3 "Ensure healthy lives and promote well-being for all at all ages" included in sustainable development goals (SDGs) led by the United Nations and will contribute greatly to goal target 3.3 "By 2030, end the epidemics of AIDS, tuberculosis, malaria and neglected tropical diseases and combat hepatitis, water-borne diseases and other communicable diseases".

According to the present invention it is possible to provide an inactivation method and an inactivation system that is designed to produce a desired inactivating effect with increased reliability using ultraviolet light that has an extremely small influence on the human body.

### BRIEF DESCRIPTION OF THE DRAWINGS

Fig. 1A is a schematic view showing an embodiment of an inactivation system;
Fig. 1B is a top view of a table and chairs in Fig. 1A;
Fig. 2 is an enlarged view of a vicinity of a light source device shown in Fig. 1A;
Fig. 3A is a drawing showing a human sensor detecting presence of a human around a table;
Fig. 3B is a top view of a table and chairs in Fig. 3A;
Fig. 4 is a graph showing survival rate of bacteria plotted against integrated irradiance of ultraviolet light;
Fig. 5 is a schematic view showing an embodiment of an inactivation system; and
Fig. 6 is a schematic view showing an embodiment of an inactivation system.

### DETAILED DESCRIPTION OF THE PREFERRED EMBODIMENTS

An inactivation method and an inactivation system according to the present invention will be described below with reference to the drawings. It is to be noted that all the drawings referred to below regarding the inactivation system are schematic illustrations, and dimensional ratios and numbers of parts on the drawings do not necessarily match the actual dimensional ratios and numbers of parts.

### [Inactivation system]

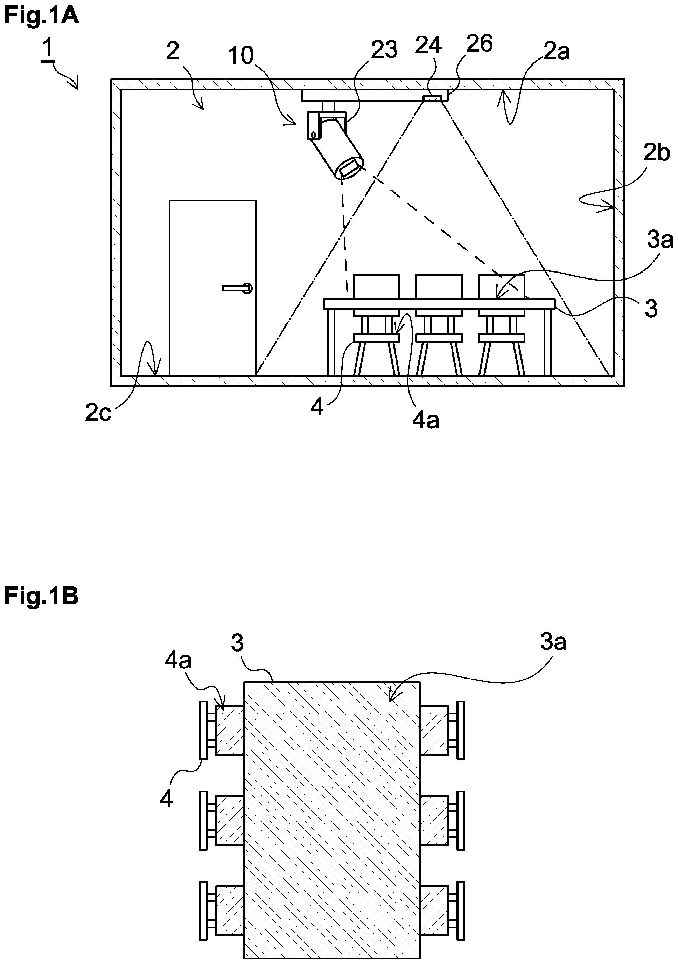

Fig. 1A is a schematic view showing an embodiment of an inactivation system 1, and Fig. 1B is a top view of a table 3 and chairs 4 in Fig. 1A. In the inactivation system 1 of the present embodiment, as shown in Fig. 1A, a light source device 10 fixed to a ceiling 2a inside a space 2 is configured to inactivate pathogens on an upper surface 3a of the table 3 and a seat surface 4a of the chair 4 that are each an object to be treated disposed inside the space 2. The light source device 10 may be fixed to a place, such as an inner wall surface of the space 2, other than the ceiling, or may be configured to be detachable from any place inside the space 2.

An inorganic antibacterial agent in the shape of a sheet is stuck on each of the upper surface 3a of the table 3 and the seat surface 4a of the chair 4 to form an antibacterial layer. In the inorganic antibacterial agent, an inorganic compound, zeolite, supports a specified substance, silver, that is displaying antibacterial and antiviral characteristics. In Fig. 1B, the antibacterial layer is illustrated by hatching. The antibacterial agent used to introduce a specified substance displaying at least one of antibacterial and antiviral characteristics (hereinafter simply referred to as a "specified substance") to the upper surface 3a of the table 3 and the seat surface 4a of the chair 4 may be an organic antibacterial agent. If a substance equivalent to the specified substance is already present on the upper surface 3a and the seat surface 4a, such as a case in which a member constituting of a top plate of the table 3 and the seat surface 4a of the chair 4 is made of silver, the specified substance may not be additionally anchored using an antibacterial agent.

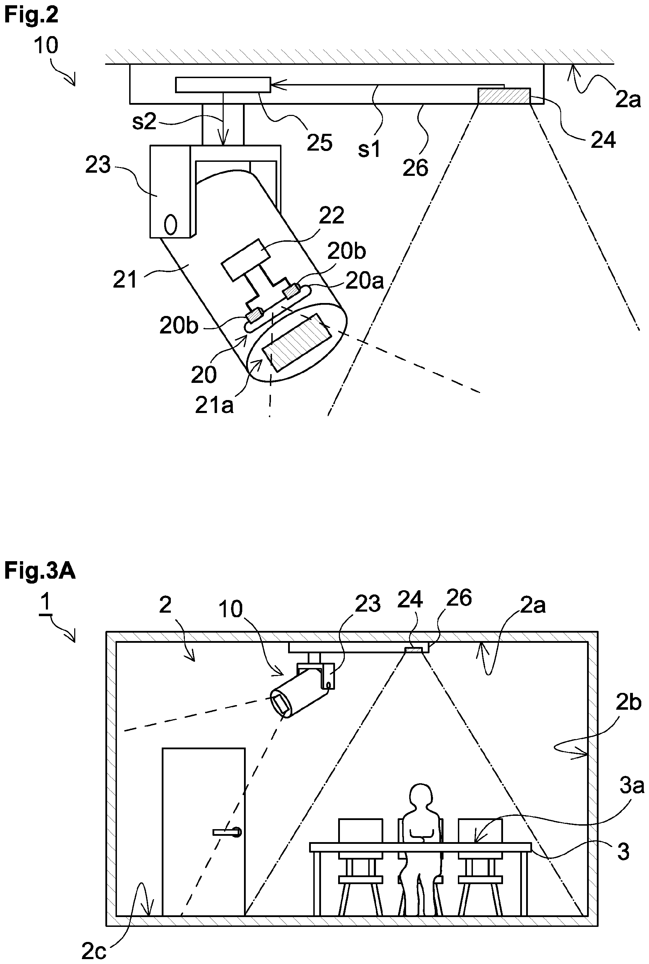

Fig. 2 is an enlarged view of a vicinity of the light source device 10 shown in Fig. 1A. As shown in Fig. 2, the light source device 10 includes an excimer lamp 20, a housing 21, a power supply unit 22, a drive unit 23, a human sensor 24, and a controller 25. In Fig. 2, the excimer lamp 20, the power supply unit 22, the human sensor 24, and the controller 25 are each mounted inside the housing 21 or a fixing unit 26 are illustrated for the convenience of explanation. The power supply unit 22 and the controller 25 are schematically illustrated to show that these parts are included in the light source device 10.

As shown in Fig. 2, the excimer lamp 20 is an ultraviolet light source that includes a light-emitting tube 20a and a pair of electrodes (20b, 20b) that are separated from each other along a tube axis of the light-emitting tube 20a such that the light-emitting tube 20a is placed on the pair of the electrodes. A voltage is applied between the pair of the electrodes. A light-emitting gas that contains krypton (Kr) and chlorine (Cl) is sealed inside the light-emitting tube 20a. When a voltage is applied between the pair of the electrodes (20b, 20b), an electric discharge occurs inside the light-emitting tube, causing ultraviolet light with a peak wavelength of about 222 nm to be emitted. The "about" described herein is intended to permit the possibility of an error of ± 2 nm in peak wavelength due to an individual difference caused by partial pressure of the inner gas or a secular change associated with usage.

In the present embodiment, the excimer lamp 20 may have a structure, for example, what is called a single tube shape or a double tube shape, or what is called a flat tube shape. A cross section of the flat tube-shaped structure cut along a plane orthogonal to the tube axis is rectangular.

The excimer lamp 20 may have any configuration that enables emission of ultraviolet light with a wavelength within a range of 190 nm or more and less than 240 nm and may be, for example, an excimer lamp that includes the light-emitting tube 20a in which a light-emitting gas containing krypton (Kr) and bromine (Br) is sealed and that emits ultraviolet light with a peak wavelength of about 207 nm. The ultraviolet light source mounted in the light source device 10 may be any light source such as an LED, with proviso that the light source can emit ultraviolet light having a wavelength in the wavelength range described above.

In the present embodiment, lighting of the excimer lamp 20 is controlled by the controller 25 such that the irradiance on the upper surface 3a of the table 3 is 10 µW/cm². In addition to ability to inactivate pathogens on the upper surface 3a of the table 3 and the seat surface 4a of the chair 4 and to prevent the integrated irradiation dose from exceeding the TLV and ensure safety even if a human is irradiated with the ultraviolet light, the irradiance of ultraviolet light on the upper surface 3a of the table 3 or the seat surface 4a of the chair 4 is preferably controlled to be less than or equal to 5 µW/cm² and is more preferably controlled to be less than or equal to 1 µW/cm².

The housing 21 is a component that houses the excimer lamp 20 inside and as shown in Fig. 2, includes a transmissive window 21a formed to extract ultraviolet light emitted from the excimer lamp 20 out of the housing. A material that the transmissive window 21a is made of is quartz glass, for example.

Although not shown in the drawings, in the present embodiment, the transmissive window 21a includes an optical filter formed to transmit at least part of ultraviolet light with a wavelength within a range of 190 nm or more and less than 240 nm and prevent transmission of ultraviolet light with a wavelength of 240 nm or more. If light intensities in a wavelength range of 240 nm and more of the ultraviolet light emitted from the excimer lamp 20 are extremely low and do not possibly cause an adverse effect on the human body, an optical member such as the optical filter may not be formed and the transmissive window 21a may be simply an opening.

The power supply unit 22 is an electric circuit electrically connected with the pair of the electrodes (20b, 20b) of the excimer lamp 20 to generate an alternating voltage applied between the electrodes (20b, 20b).

The drive unit 23, as shown in Fig. 2, is a member for connecting the fixing unit 26, which fixes the light source device 10 to the ceiling 2a, to the housing 21 and changes an orientation of the transmissive window 21a of the housing 21, i.e., a light emission direction of the light source device 10, in response to a driving signal s2 output from the controller 25.

In the present embodiment, the human sensor 24 is an infrared sensor mounted in the fixing unit 26 such that a detection area covers a space between the light source device 10 and the table 3 and the chairs 4. The human sensor 24 corresponds to a "human detector" (refer to Fig. 1A). The human sensor 24, as shown in Fig. 2, outputs a human detection signal s1 to the controller 25 when detecting presence of a human between the light source device 10 and the antibacterial layer formed on the seat surface 4a of the chair 4.

The human sensor 24 may be disposed in the housing 21 although the human sensor, as shown in Fig. 2, is mounted in the fixing unit 26 of the light source device 10. The human sensor 24 may be disposed on the ceiling 2a, an inner wall surface 2b, or another area in the space 2 to be separate from the light source device 10. Besides the human sensor 24 such as an infrared sensor or a distance sensor, a camera with a face recognition function, for example, may be used as a human detector.

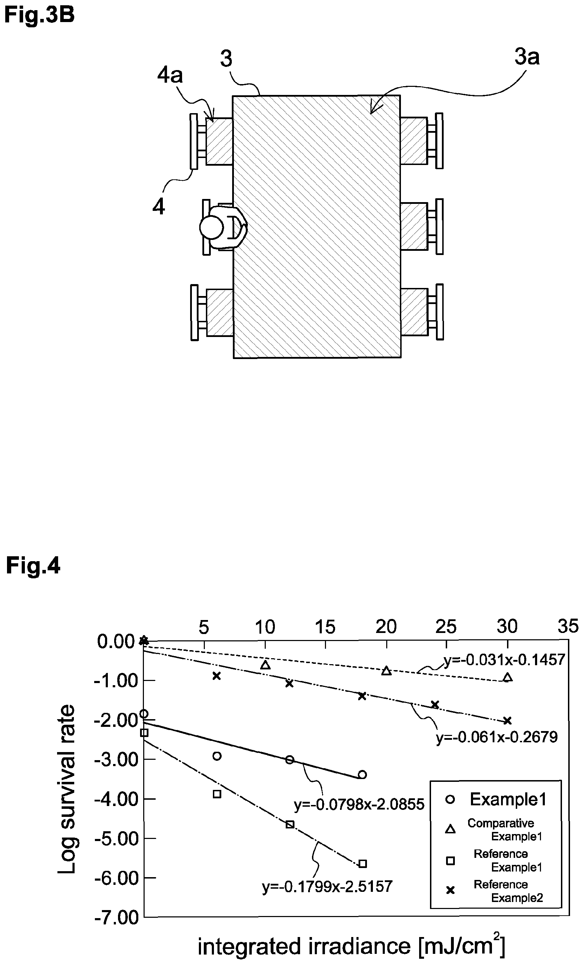

When the human leaves the chair 4 and the human detection signal s1 is not output from the human sensor 24, the controller 25 outputs the driving signal s2 to the drive unit 23 to cause the light source device 10 to emit the ultraviolet light toward the upper surface 3a of the table 3 and the seat surface 4a of each chair 4. Fig. 3A is a drawing showing the human sensor 24 detecting presence of a human. Fig. 3B is a top view of the table 3 and the chairs 4 in Fig. 3A, viewed from the light source device 10.

In the present embodiment, as shown in Figs. 3A and 3B, when the human sensor 24 detects the presence of a human between the light source device 10 and the upper surface 3a of the table 3 or the seat surface 4a of any chair 4, the light source device 10 operates to emit the ultraviolet light in a direction that is not aimed at the antibacterial layer (a hatched area in Fig. 3B) formed on each of the upper surface 3a of the table 3 and the seat surface 4a of the chair 4.

The light source device 10 may be configured to avoid irradiation of the human with the ultraviolet light as much as possible, for example, by being controlled to narrow a zone of the ultraviolet irradiation when the human sensor 24 detects the presence of a human between the light source device 10 and the upper surface 3a of the table 3 or the seat surface 4a of any chair 4.

The controller 25 may be a central processing unit (CPU) or a microprocessor unit (MPU) mounted inside the housing 21 or may be an external device, such as a smartphone, a tablet computer, or a PC, connected to the light source device 10 by wired or wireless communications.

The inactivation system 1 of the present embodiment, with the configuration and control described above, inactivates pathogens by irradiating the upper surface 3a of the table 3 and the seat surface 4a of each chair 4, to which the specified substance is anchored, with the ultraviolet light having a wavelength of 222 nm under a condition in which any human is not present between the light source device 10 and the antibacterial layer.

### [Verification experiment]

A verification experiment was conducted to examine an inactivating effect produced by irradiating the surface of an object on which a specified substance and a stain were present with ultraviolet light having a wavelength within a range of 190 nm or more and less than 240 nm. Details of the verification experiment will now be described.

### (Example 1)

In Example 1, an antibacterial sheet was attached to a surface of a stainless steel-made plate (hereinafter referred to as a "SUS plate"), a solution constituting a stain model was dropped to put the SUS plate in a clean state. After that, the main surface of the SUS plate was irradiated with ultraviolet light having a wavelength of 222 nm. Specifically, integrated irradiance (an integrated light amount) described later was adjusted by changing irradiation time with irradiance of the ultraviolet light set at 0.1 mW/cm². The antibacterial sheet used for the plate was TOLI Corporation's made Cushioned Floor (CF sheet CF9469), which contained silver as a specified substance.

### (Comparative Example 1)

Conditions in Comparative Example 1 were similar to those in Example 1 except that the antibacterial sheet was not attached.

### (Reference Example 1)

Conditions in Reference Example 1 were similar to those in Example 1 except that the solution constituting a stain model was not dropped and the SUS plate in a no-load state was irradiated with the ultraviolet light.

### (Reference Example 2)

Conditions in Reference Example 2 were similar to those in Example 1 except that the antibacterial sheet was not attached, the solution constituting a stain model was not dropped, and the SUS plate in a no-load state was irradiated with the ultraviolet light.

### (Verification method)

This verification was conducted using Staphylococcus aureus. The EN test method which is a basic test method for evaluating bactericidal activity specifies a clean state as a state indicating a contamination level assumed in a target region. The clean state corresponds to the state in which a load substance is added to simulate a contaminated state. In this verification, the survival rate of Staphylococcus aureus against integrated irradiance was examined in the clean state and in a no-load state (ideal state) in which any load substance is not added at all.

As a sample in the clean state, a sample prepared by adding, as a model stain, a protein (bovine serum albumin (BSA)) to a Staphylococcus aureus solution and dropping the solution on the SUS plate was used. An amount of the BSA and an amount of the Staphylococcus aureus used to prepare the sample in the clean state were 0.3% by mass and 1,385,000 CFU per SUS plate, respectively. CFU stands for colony forming unit.

### (Results)

Fig. 4 is a graph showing the survival rate of the bacteria plotted against integrated irradiance of the ultraviolet light in this verification. The vertical axis represents the survival rate on a logarithmic scale, and the horizontal axis represents integrated irradiance on the SUS plate. The graph of Fig. 4 shows approximate curves along points plotted for the respective examples and equations for the approximate curves.

First, as shown in Fig. 4, results in Example 1 and Reference Example 1 show greater progress in inactivation (the intercept being far apart from 0) even at an integrated irradiance of 0 mJ/cm² due to an inactivating effect of the antibacterial sheet compared to results in Comparative Example 1 and Reference Example 2.

The graph shown in Fig. 4 proves that the plotted line for Example 1 in which the antibacterial sheet is attached to the surface of the SUS plate has an inclination about 2.6 times larger than that for Comparative Example 1 in which the antibacterial sheet is not attached. This means that the sterilization effect in Example 1 is higher than that in Comparative Example 1 even when the plate is irradiated with ultraviolet light in the same irradiation conditions.

Similarly, the graph proves that the plotted line for Reference Example 1 in which the antibacterial sheet is attached to the surface of the SUS plate has an inclination about 3.0 times larger than that for Reference Example 2 in which the antibacterial sheet is not attached.

Example 1 and Reference Example 1 show the higher inactivating effect than Comparative Example 1 and Reference Example 2 do. A reason for this is surmised that a silver ion derived from the silver contained in the antibacterial sheet is irradiated with the ultraviolet light and the silver ion is thereby excited, displaying an increased antibacterial characteristic against the Staphylococcus aureus.

According to the results of Example 1 and Reference Example 1, due to the presence of the specified substance on the surface of the object to be treated, the survival rate is approximately -2 Log (about 1/100) without ultraviolet irradiation (at an integrated irradiance of 0 mJ/cm²). It can be said that this is caused by the inactivating effect of the antibacterial sheet.

In other words, it is proved that irradiating the surface of the object, on which the specified substance is present, with the ultraviolet light produces an increased inactivating effect compared to the conventional technique due to the inactivating effect of the specified substance and excitation of the silver ion, a specified substance derived from silver, by the ultraviolet light.

In view of the reason for an improvement in inactivating effect caused by irradiation of the antibacterial sheet, which contains silver as a specified substance, with the ultraviolet light, it is surmised that the inactivating effect of even an antibacterial sheet (antibacterial agent) containing a metal ion such as a copper ion or a zinc ion is similarly improved by ultraviolet irradiation.

Thus, the specified substance is any substance that displays an antibacterial or antiviral characteristic. Specifically, the specific substance is preferably made of a metal ion or a metal compound that contains at least one metal component selected from the group consisting of silver, copper, zinc, platinum, nickel, cobalt, lead, iron, calcium, sodium, aluminum, and manganese. The specific substance is more preferably made of a metal ion or a metal compound that contains any metal component selected from silver, copper, and zinc among the metals above. The specific substance is particularly preferably made of a metal ion or a metal compound that contains a silver component.

The inactivation system 1 having the configuration described above enables inactivation of bacteria or viruses on the object the surface of which a stain is adhering to by using the ultraviolet light that has an extremely small influence on the human body without unnecessarily improving irradiance of the light on the surface of the object.

When the human sensor 24 detects presence of a human between the light source device 10 and the antibacterial layer, the inactivation system 1 having the configuration described above emits the ultraviolet light in a changed direction that is not aimed at the area on which the antibacterial layer is formed. This suppresses irradiation of the human with the ultraviolet light and thus provides improved safety.

In the inactivation system 1, the controller 25 may be configured to output a control signal to the power supply unit 22 to decrease or stop the voltage applied to the electrodes (20b, 20b) of the excimer lamp 20 and decrease the intensity of the emitted ultraviolet light or stop emission of the light when the human sensor 24 detects the presence of a human between the light source device 10 and the upper surface 3a of the table 3 or the seat surface 4a of any chair 4. This configuration also avoids irradiation of the human with the ultraviolet light at a high irradiance required for inactivation and thus provides increased safety.

On condition that humans are less likely to be irradiated with the ultraviolet light at high irradiance in such cases when the inactivation system 1, for example, includes a timer and is configured to perform ultraviolet irradiation only in the night during which hardly anyone is coming and going, the inactivation system 1 may not include the human sensor 24.

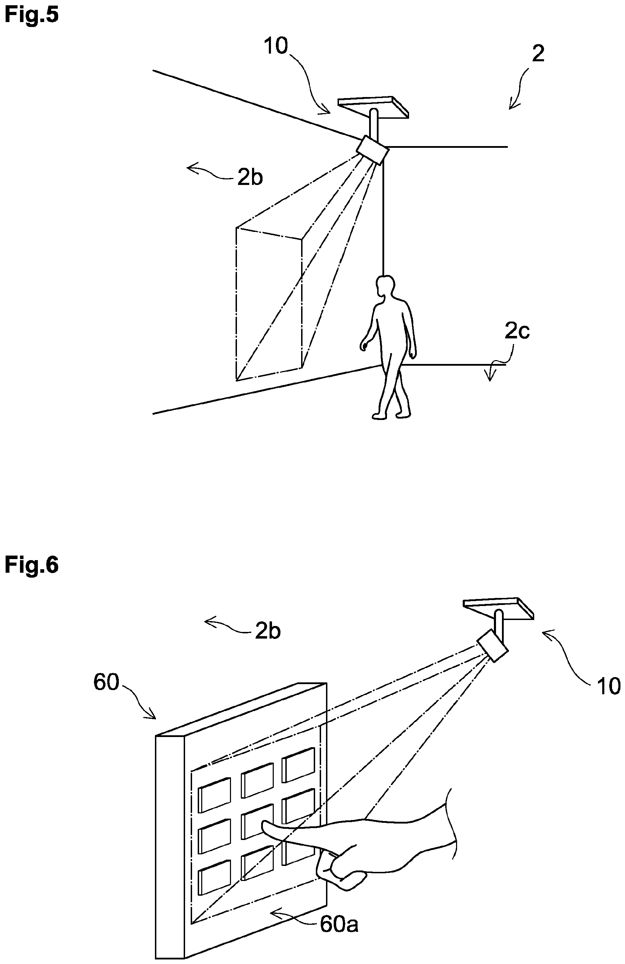

Figs. 5 and 6 are schematic views each showing an embodiment of an inactivation system 1 different from that in Fig. 1A. In the inactivation system 1, as shown in Fig. 5, the light source device 10 may be configured to emit ultraviolet light toward the inner wall surface 2b on which an antibacterial layer (not shown) is formed in the space 2. An object to be treated for inactivation may be a floor surface 2c rather than the inner wall surface 2b.

The inactivation system 1, as shown in Fig. 6, may be configured to inactivate pathogens on an operation surface 60a of an operation panel 60 (for example, of an air conditioner or another apparatus) that is mounted on the inner wall surface 2b and that is frequently touched by humans. A button for an elevator, a doorknob, or the like may be an object to be treated for inactivation. In any case, if a specified substance is not present on the surface, a step of anchoring a specified substance to the surface, which is irradiated with the ultraviolet light, is performed.

The inactivation system 1 of the present embodiment is configured to irradiate any of the upper surface 3a of the table 3 and the seat surface 4a of each chair 4, on which the antibacterial layer is formed in advance, with the ultraviolet light. Further, a specified substance may be anchored to other surfaces that are irradiated with the ultraviolet light, such as the surface of an object, the inner wall surface 2b, or the floor surface 2c in the space 2, to improve the inactivating effect.

The configurations of the inactivation systems 1 described above are merely examples, and the present invention is not limited to the illustrated configurations.

## Claims

1. A method for inactivating bacteria or viruses in a space that is defined, the method comprising a step (A) of irradiating of an inner wall surface, a floor surface in the space or a surface of an object disposed in the space where a specified substance displaying at least one of antibacterial and antiviral characteristics is present with ultraviolet light having a wavelength within a range of 190 nm or more and less than 240 nm.

2. The method for inactivating bacteria or viruses, according to claim 1, wherein the step (A) is performed by anchoring the specified substance to any of the inner wall surface, the floor surface, and the surface of the object to be treated in a state of being irradiated with the ultraviolet light in the space.

3. The method for inactivating bacteria or viruses, according to claim 1, wherein the step (A) is performed after the specified substance is anchored to any of the inner wall surface, the floor surface, and the surface of the object to be treated in the space by irradiating an area to which the specified substance is anchored with the ultraviolet light.

4. The method for inactivating bacteria or viruses, according to claim 2 or 3, wherein an inorganic antibacterial agent containing the specified substance supported by an inorganic compound is stuck on any of the inner wall surface, the floor surface, and the surface of the object to be treated in the space to anchor the specified substance to any of the inner wall surface, the floor surface, and the surface of the object.

5. The method for inactivating bacteria or viruses, according to claim 4, wherein the specified substance contained in the inorganic antibacterial agent is made of a metal ion or a metal compound that contains at least one metal component selected from the group consisting of silver, copper, zinc, platinum, nickel, cobalt, lead, iron, calcium, sodium, aluminum, and manganese.

6. A system for inactivating bacteria or viruses on an object to be treated in a space that is defined, the system comprising:
an antibacterial layer formed on a surface of the object, the antibacterial layer containing a specified substance that displays at least one of antibacterial and antiviral characteristics; and
a light source device mounted on a ceiling or a wall surface in the space to emit ultraviolet light with a wavelength within a range of 190 nm or more and less than 240 nm toward the antibacterial layer on the object.

7. The system for inactivating bacteria or viruses, according to claim 6, comprising:
a human detector to detect whether or not a human is present between the light source device and the antibacterial layer;
a drive unit to change a light emission direction of the light source device; and
a controller to control the drive unit in response to a human detection signal output from the human detector,
wherein when the human detector detects presence of a human, the controller controls the drive unit to cause the light source device to emit the ultraviolet light in a direction that is not aimed at the object.

8. The system for inactivating bacteria or viruses, according to claim 6, comprising:
a human detector to detect whether or not a human is present between the light source device and the antibacterial layer; and
a controller to control intensity of the ultraviolet light emitted from the light source device in response to a human detection signal output from the human detector,
wherein when the human detector detects presence of a human, the controller controls the light source device to decrease the intensity of the emitted ultraviolet light or stop emission of the ultraviolet light.
